(19) **Europäisches Patentamt**
**European Patent Office**
**Office européen des brevets**

(11) **EP 3 101 574 A1**

(12) **EUROPEAN PATENT APPLICATION**

(43) Date of publication:
**07.12.2016 Bulletin 2016/49**

(51) Int Cl.:
**G06F 19/28** *(2011.01)*    **G06F 17/30** *(2006.01)*

(21) Application number: **15170816.1**

(22) Date of filing: **05.06.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Limbus Medical Technologies GmbH 18055 Rostock (DE)**

(72) Inventor: **Liesfeld, Ben, Dr. 18055 Rostock (DE)**

(74) Representative: **Pfenning, Meinig & Partner mbB Patent- und Rechtsanwälte Joachimsthaler Straße 12 10719 Berlin (DE)**

(54) **DATA QUALITY MANAGEMENT SYSTEM AND METHOD**

(57)     The subject matter presently claimed relates to a data quality management system and method whereby a first data point comprising a first obtained data and a first assigned value from is received from a first data repository (101), a first quality score as well as a first storable data of the first data point is determined and/or stored. A second data point comprising a second obtained data, which is similar to the first obtained data according to a predefined similarity measure, and a second assigned value is received from the second data repository (102), a second quality score as well as a second storable data is determined from the second data point and/or stored and a second transmittable data, determined from the second data point and/or the second quality score is transmitted to the first data repository (101), causing the first data repository (101) to re-evaluate the first assigned value.

Fig. 3

## Description

The subject matter of this application relates to a system and a method for data quality management as well as a method for improving data quality of a data repository, wherein the former and the latter methods relate to a plurality of inter-related methods forming a single inventive concept.

Modern technologies allow to rapidly identify and quantify molecules in samples of organic tissue. Examples for these technologies are mass spectrometry and DNA sequencing. The process of identification and quantification has been greatly accelerated and become more and more efficient and therefore also cheaper. This development has reached the point where it appears more appropriate to perform molecular analysis first and then develop a hypothesis about causalities than the other way around. Typically, a large amount of data is collected first and then correlations are examined using statistical approaches.

In general, biological systems are very complex. Thus, the number of biological samples which can be examined or the number of meaningful data points which can be extracted from the sample may be too small to draw reliable conclusions. The population from which the samples are drawn may also be limited or biased which impacts the interpretation of the data points. Therefore, the systematic and structured collection of context information about the biological organism is crucial in order to perform an interpretation of the statistical data.

Data objects in biological research or diagnostics and their values, including e.g. assessments by an expert, are not static, they are rather volatile and are continuously re-assessed and re-classified. One of the reasons is that causality between parameters can rarely be determined, therefore correlations are the basis of assessment and classification. Correlations may change over time and are corroborated with every additional case, patient, sample, or other piece of context information that contributes to the assessment. Therefore an assessment of biological data often takes place where data is collected, because typically the greatest human expertise is located there. Data models and ontologies change rather rapidly over time due to the fast-paced progress in biological and related sciences and in their related technical fields. Standards - if existent - are often quickly outdated and neglected.

Due to the complexity of the task the assessment by human experts is often considered superior to computed predictions. Still, two different parties of experts may arrive at different conclusions, even though both parties followed the same formal rules during data collection, so that data compliant with the same formal requirements is available to each party. The two parties are looking at different sub-samples from the overall population, and may therefore arrive at different conclusions, for example caused by different past experiences.

The state of the art, US8359297, describes receiving conflicting data values from multiple sources for a data element, using a conflict rule to determine the main data value for the data element, which is subsequently stored for use. Therefore complete data sets from many sources are received and one main data store is created that contains a complete and consolidated set of data.

The state of the art does not address the issue that ownership of the data may not reside with a single entity so that it may not be possible to store all data in a central repository. Furthermore, data may be submitted to confidentiality which may also prevent data from being stored in a central repository, this applies to patient or clinical data in particular and the plurality of repositories may be operating independently and may not e. g. agree on a specific set of rules to resolve data conflicts. Each data repository may have its own specific rules for conflict resolution.

It is, therefore, an object of the present subject matter to provide a data quality management system and method in addition to a method for automatically improving data quality of a computer-implemented data repository. The claimed system and methods automatically improve the data quality as opposed to just determining and monitoring data quality.

The method for automatic data quality management and the method for automatically improving the data quality of a computer-implemented data repository relate to a plurality of inter-related methods for improving data quality. The two methods describe the two opposing sides of an interface for automatic data transfer and, thus, form a single inventive concept.

This is achieved by the presently claimed data quality management system as well as the presently claimed data quality management method as well as the presently claimed method for automatically improving the data quality of a computer-implemented data repository. Advantageous embodiments of the subject matter presently claimed are further disclosed in the dependant claims.

The data quality management system according to the subject matter presently claimed comprises a central computing component as well as computer-implemented data transmission connections to a first and a second computer-implemented data repository stored on at least one database server.

The central computing component is implemented on a computing device which comprises a computer-implemented data storage module, for example a data base, a computer-implemented data communication module and a computer-implemented quality score module.

The central computing component is configured to receive, via the communication module, a first data point comprising a first obtained data and a first assigned value from the first data repository. Then, the central computing component is configured to determine, in the quality score module, a first quality score of the first data point, to further determine a first storable data, which is determined from the first data point and/or the determined first quality score, and to store the first storable data in the storage module.

The central computing component is further configured to receive, via the communication module, a second data point comprising a second obtained data, which is similar to the first obtained data according to a predefined similarity measure, and a second assigned value from the second data repository. Then, the central computing component is configured to determine, in the quality score module, a second quality score of the second data point, to further determine a second storable data from the second data point and/or the second quality score and to store the second storable data in the storage module.

The central computing component is further configured to determine a second transmittable data from the second data point and/or the second quality score and to transmit the second transmittable data to the first data repository, causing the first data repository to re-evaluate the first assigned value.

The first and second obtained data can, for example, be measured and/or experimental data, data which has been collected automatically and/or electronically or entered manually. The first and/or second obtained data can, for example, relate to biomedical data or genetics. The obtained data as well as the assigned values may further comprise information about how the obtained data was obtained, about the number of samples used in determining the respective first and/or second assigned value and/or about the level of certainty with which the assigned values were assigned.

The first and/or second assigned values may have been assigned automatically, by an algorithm, through a statistical learning process or manually by an expert evaluating the obtained data.

Determining of the first and/or second quality score may, for example, be based only on the metadata received from the first and/or second data repository, including metadata about how many samples of a specific obtained data the assigned value was based on or about the method used for collecting the obtained data and/or for assigning the assigned value.

The first and the second obtained data may be considered similar according to a similarity measure, also referred to as matching, if they, for example, contain overlapping data, if a portion of the first and/or second data is identical, if the first and second obtained data has been obtained from the same source or sample and/or if the first and second obtained data is identical.

The storable first and/or second data may contain any subset of the first or second, respectively, data point as well as the respective quality score. In particular, the storable data may contain a data point identifier, a history of quality scores of the obtained data, information about the data model and about model transformation, information about metadata relating to the data point including, e.g. the number of variants in the respective data repository or the number of updates of the assigned value, and/or a history of the respective quality scores. Preferably, the first and/or second storable data contains at least a data identifier, including information about the respective data repository.

The algorithm used by the quality score module to determine a quality score may further be based on the number of data points which were already evaluated, i.e. to which a quality score has already been assigned, by the quality score module. It is, thus, possible to re-evaluate and/or change the quality scores which were already assigned by the quality score module after a specified time has passed or after a specified number of data points, preferably data points with similar obtained data, have been evaluated.

The central computing component may further be configured to transmit the second transmittable data, which may contain a subset of the available data determined and containing information in the same manner as the storable data, to the first data repository causing the first data repository to update the first assigned value. Preferably, the first assigned value is updated to an updated first assigned value which is different from the first assigned value, preferably in such a way that the quality of the updated first assigned value is improved for future processing.

Transmitting the second transmittable data to the first data repository, causing the first data repository to update the first assigned value is particularly important when data relating to similar subject matter is collected and evaluated, i.e. values are assigned to the collected or obtained data, by several different entities, possibly using different collection and/or value assignment schemes.

Updating, changing and/or improving the assigned value of a data point stored in the first data repository on the basis of the assigned value and/or metadata of a second, similar data point stored in a second data repository, provides an opportunity for creating improved, more consistent data collections while preserving energy needed for collecting further samples by each individual entity. The updated and/or improved data may then be used in practical applications, leading to improved results. For example, the updated and/or improved data may be used as an input for an automated, clinical and/or industrial process.

The central computing component may further be configured to receive an updated first data point comprising the first obtained data and an updated first assigned value from the first data repository. The central component may then be configured to determine, in the quality score module, an updated first quality score of the updated first data point, to determine an updated first storable data from the updated first data point and/or the updated first quality score and to store the updated first storable data in the storage module. Further, the central component may be configured to transmit the updated first quality score to the first and/or second data repository via the computer-implemented data communication module.

The system for data quality management may further comprise a computer-implemented model-transformation module, configured to transform data from a first data format into a second data format. In particular, when the first data repository contains data stored in the first data format and the second data repository contains data stored in the second data format, the central component may be configured to transform, in the model transformation module, the data received from the first data repository into the second data format, the data received from the second data repository into the first data format and/or the data received from the first and/or the second data repository into a central data format.

As the first and the second data repositories may belong to and/or be administered by different entities, the first and second data points may be stored in different and/or incompatible data formats. Thus, the model transformation unit may allow a comparison of data points relating to similar obtained data, even if the data points are stored in different data formats.

Furthermore, the system for data quality management may comprise the first and/or second data repository, wherein each of the respective data repositories may comprise a communication module, which serves as an interface, a storage module and/or a metadata module.

The metadata module serves to determine metadata, i.e. data describing the actual data, from the actual data stored in the data repository. Metadata may, for example, contain information about a number of samples, how data was collected and/or how data has changed over time. In data repositories containing personal and/or confidential information, metadata may serve to anonymise the data prior to submitting it to a different data processing device.

The presently claimed method for automatic data quality management comprises the following steps, which are implemented to be executed on a computer processor:

- receiving a first data point comprising a first obtained data and a first assigned value from a first data repository,
- determining a first quality score of the first data point,
- determining a first storable data from the first data point and/or the first quality score,
- storing the first storable data in a computer implemented central storage module,
- receiving a second data point comprising a second obtained data, which is similar to the first obtained data according to a predefined similarity measure, and a second assigned value from a second data repository,
- determining a second quality score of the second data point,

- determining a second storable data from the second data point and/or the second quality score,
- storing the second storable data in the storage module and
- transmitting a transmittable second data determined from the second data point and/or the second quality score to the first data repository causing the first data repository to re-evaluate the first assigned value.

The step of transmitting a transmittable second data to the first data repository, may in particular, cause the first data repository to update the first assigned value. Preferably, the updated first assigned value may be different from the first assigned value.

The further advantageous and possible characteristics of the first, second and/or updated data points, obtained data, assigned value, quality scores and/or storable data as described above with respect to the claimed system also apply to the claimed method for automatic data quality management.

The method for automatic data quality management may further comprise the following steps:

- receiving an updated first data point comprising the first obtained data and an updated first assigned value from the first data repository,
- determining an updated first quality score of the updated first data point,
- determining an updated first storable data from the updated first data point and/or the updated first quality score, and
- storing the updated first storable data in the central storage module.

Additionally, the method for automatic data quality management may comprise the step of transmitting the updated first quality score to the first and/or second data repository.

Furthermore, the first and/or second obtained data of the data quality management system and/or the method for automatic data quality management may preferably comprise biological, medical, genetic and/or genomic data. Biological and medical data may comprise information about the existence of or the amount or the concentration of specific molecules or molecular fragments in biological samples. Medical information may also comprise descriptions of physiological features and pathological information. Genetic and genomic data may comprise information about existence or non-existence of specific structural features or genetic sequences in genetic information derived from biological samples.

Preferably the presently claimed method is used in a computer program product for data quality management which is stored on a computer-readable medium and which, when run on a computer, is configured to execute the method for data quality management as described above.

The presently claimed method for automatically improving data quality of a computer-implemented data repository involves the following steps:

- transmitting a first data point comprising a first obtained data and a first assigned value to a central computing component
- receiving information about a second data point comprising a second obtained data, which is similar to the first obtained data according to a predefined similarity measure, and a second assigned value from the central computing component
- re-evaluating the first assigned value on the basis of the received information about the second data point.

Re-evaluating the first assigned value may comprise automatically updating, changing and/or improving the first assigned value.

The method for improving data quality of the computer-implemented data repository may further comprise the step of determining a quality score of a data point stored in the data repository or received from a central computing or another data repository.

Determining the quality score may also happen within a data repository specific quality score module, independently of a quality score determined by the central communication module. This may be advantageous if the data repository wants to implement a quality standard different from that of the central computing component or if the data stored in the data repository as well as the metadata obtained from the data is confidential.

The further advantageous and possible characteristics of the first, second and/or updated data points, obtained data, assigned value, quality scores and/or storable data as described above with respect to the claimed system also apply to the claimed method for automatic data quality management.

The presently claimed data quality management system may further comprise at least one of a first and/or a second computer-implemented data repository interface which is configured to be run on a data base server. The first and/or second data repository interface may be configured according to the method for improving data quality of the computer implemented data repository as described above.

Exemplary embodiments of the subject matter presently claimed are described below referring to the following Figures, where

Fig. 1    shows a schematic view of a network consisting of a central computing component, several data repositories and a reader terminal,

Fig. 2    shows the subcomponents of the central computing component and the data repository,

Fig. 3    depicts a flowchart indicating the steps performed when the central component receives data from a repository,

Fig. 4    depicts a flowchart indicating a data review process performed by the data repository,

Fig. 5    shows a flowchart indicting the different steps for a distributed calculation of quality scores, and

Fig. 6    shows a flowchart for mediating conflict resolution.

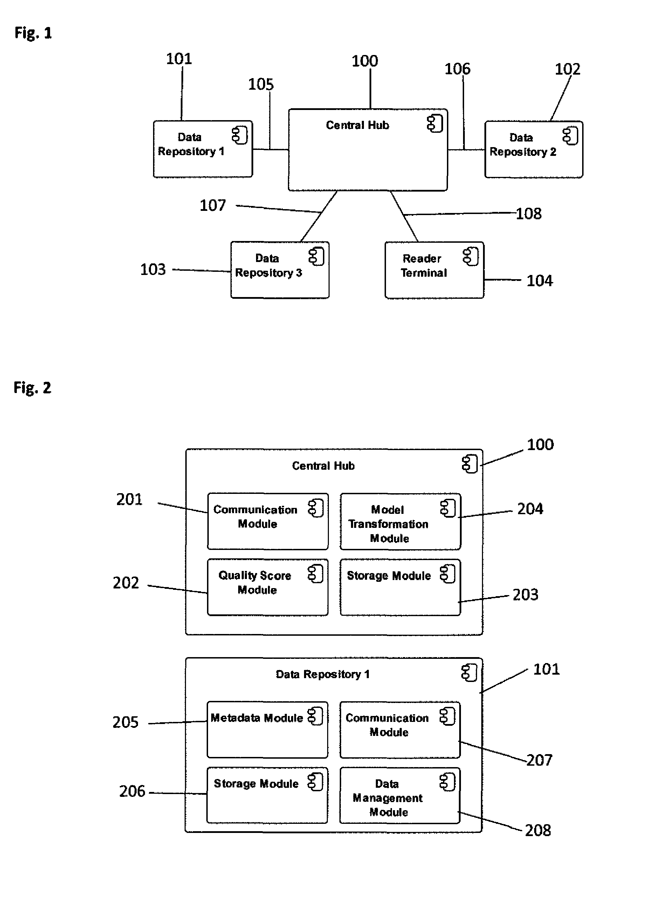

Fig. 1 shows a schematic view of a system for data quality management according to an exemplary embodiment, comprising a central computing component 100, also referred to as a central hub component or hub, which provides interfaces and data transmission connections 105, 106, 107 to entities each of which comprises a biological reference data repository 101, 102, 103. In the following the entirety of central hub component 100 and interfaced data repositories 101, 102, 103 will be called "network". The data models and ontologies of the data repositories 101, 102, 103 may be different to each other.

In another embodiment of the claimed subject matter, as also shown in Fig. 1, the central component 100 may not only maintain interfaces to data repositories 101, 102, 103 but also to maintain a data transmission connection 108 to at least one reader terminal 104 that retrieves data from the central component 100 and does not consist of large data repositories itself.

In a favourable embodiment of the claimed subject matter, as shown in Fig. 2, the central hub component 100 consists of subcomponents like a communication module 201 that performs communication with data repositories 101, 102, 103 and reader terminals 104, a quality score module 202 which performs computation of quality scores, a storage module 203 which is used to commit data like quality scores to a non-transient storage and a model transformation module 204.

As also shown in Fig. 2, data repositories 101, consist of a storage module 206 which is used to store the biological reference data, a metadata module 205 which computes metadata from the data in the storage module 206 and a communication module 207, which serves as an interface for data exchange, and a data management module 208.

According to an embodiment, data repositories 101, 102, 103 and the central hub component 100 are connected via TCP/IP, their APIs are exposed via HTTP endpoints, and they may offer additional dedicated interfaces for messaging (e.g. AMPQ, the Advanced Message Queuing Protocol). Both the data repository 101, 102, 103 and the hub 100 may initiate a communication. Communication between components is encrypted via SSL (i.e., HTTPS and AMPQ+SSL are used). Additional network security measures may consist in setting up Virtual Private Networks (VPNs) for specific data repositories 101, 102, 103, to provide an additional layer of security. Storage module 206 may consist of one or more relational databases (RDBMS, using SQL) or NoSQL databases consisting of document, graph or key-value data structures.

While the basic integrity of transferred data is ensured by the lower layers in the network stack (e.g. via IP checksums), both the central hub 100 and the data repositories 101, 102, 103 may run continuous monitoring and validation services ("watchdogs") to check for inconsistencies and quality of service (e.g. the timely propagation of updated information) at runtime.

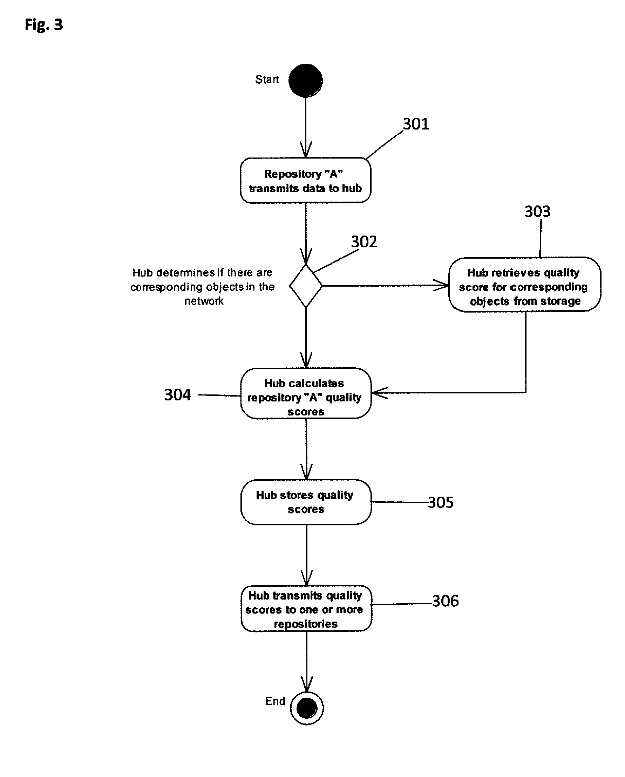

As shown in Fig. 3, a data repository "A" 101 transmits 301 data point "1" consisting of a data object identifier, a data attribute, e.g. a measured data or experimental data, and one or more metadata attributes, e.g. the number of samples, to the hub 100. The hub 100 determined 302 that there are no matching data objects in the network, calculates 304 quality scores from data point "1" and commits 305 at least some of the data to its own storage 203. Once this process has been performed at least once the hub 100 will always compare the transmitted data to the data in its storage 203 to determine if there are other matching, i.e. similar data objects in the network.

If the hub 100 now receives a data point "2", which is similar to data point "1" as the two data points contain some identical information, from a data repository "B" 102, the hub determines 302 that there is a matching data point "1" and retrieves 303 this data point and/or its quality scores from either the hub's 100 own storage 203, or from the storage 206 of the respective data repository "A" 101, computes 304 the quality scores of the transmitted data, stores 305 some of the transmitted and/or calculated data in the hub's 100 storage 203 and transmits 306 the data object identifier and the quality scores to one or more data repositories 101, 102, 103. In a favourable embodiment the data is transmitted to all repositories 101, 102, 103 that contain a matching, i.e. similar, data object. The data repositories 101, 102, 103 containing matching data objects then use the received data to re-evaluate their own data, causing data repository "A" to update and change some of the values associated with data point "1". Data repository "A" then re-sends updated data point "1" to the hub 100, causing the hub 100 to recalculate the quality score of updated data point "1".

The different components of the network, as shown in Fig. 1 and as described above, can be implemented as a computer program product for data quality management, which can be stored on at least one computer-implemented medium such as, e.g. a hard-disk drive, a CD- ROM, a DVD or any other kind of non-transient computer-readable storage. The computer program product is then configured and implemented to, when run on at least one computer, bring about the changes described in the context of the network above.

In a favourable embodiment the hub 100 stores information how data objects were re-evaluated, updated and/or changed over time by data repositories 101, 102, 103. This information may also be used to compute quality scores as described below. In one embodiment the data repositories 101, 102, 103 may initiate transfer of data to the hub 100, in another embodiment transfer may be initiated by the hub 100, e. g. in order to determine if data in data repositories 101, 102, 103 was changed or updated.

This may be illustrated with an example of data repositories 101, 102, 203 containing information about variants of the human DNA. Variants may uniquely be described by a) the coordinate in the human genome at which the change is observed and b) the observed change with respect to the reference genome. A variant may be described as "g.43076586dupT" which means that at position 43076586 in the genome the letter "T" was duplicated. In this way, variants may be identified across several different repositories.

In the storage module of the central computing component, the stored data may, in the case of this example contain the description of variants (e. g. g124566992C>T) and which repository contains information about it, the classification of the variant (benign, likely benign, unknown significance, likely pathogenic, pathogenic), all calculated quality scores for different objects like variants, submitters and/or repositories, weighting factors, a history of quality scores, per variant/per gene/per repository and/or other data, parameters for predictive powers of metadata, histories of these parameters, metadata generated during quality score calculation including the number of variants per repository and/or the number of updates over time per repository etc. (to determine the most active repository) and/or information about data models in repositories and about model transformations.

The lab that uses data repository "A" determines a new case, and thereby discovers a new variant in the sequence of the DNA of this subject. The data related to the variant contains a data attribute about its effect, e.g. this variant is "pathogenic". Repository "A" commits the data to its storage. This data will be re-used by the lab in subsequent analyses, as an in-house reference database.

The data repository "A" also transmits the identifier of the variant, a unique description based on genomic coordinates ("g.43076586dupT"), the data attribute ("pathogenic") and metadata about the variant and related information to the central hub component. The metadata may e. g. comprise information about the number of subjects that an analysis was performed with.

In the case of data repositories 101, 102, 103 containing information about the human genome similarity could mean that there is a similarity in a coordinate (position), i. e. affecting the same or a similar region and/or a similarity in the specific sequence change in a similar region, i. e. leading to the same or similar protein change, describing a similarly large deletion and/or creating a similar effect in a certain coordinate region. Generally biological, medical, genetic and or genomic data may be considered to be similar if creating or causing a similar change in an organism.

If the objects stored in the repositories are biomarkers and/or biomolecules similarity could be defined as the similarity of molecular structure. At some point one may define two different fragments A and B of a larger protein AB, measured via mass spectrometry, as evidence for the existence of the one protein AB. Concentration levels of fragments A and B may therefore be considered equivalent in determining a certain state of the human organism. Molecules may simply be called different names in different repositories.

The central component 100 receives the data and compares the data to data contained in its storage 203. This time, the hub 100 finds matching, i.e. similar, variants in its storage 203, it retrieves the data related to it, computes one or more quality scores from the data it received from repository "A" and transmits the quality scores including the scores from other repositories' data and related data attributes and metadata back to all repositories where this variant is stored. The data repositories "B" and "D" rate this variant as "benign". Repository A then displays the attributes with the highest quality score, e.g. from repository "B", as well as additional metadata from "B" (e.g. number of cases, types of analysis, other supporting evidence). As the quality scores indicate that the data from "B" is valid, repository "A" starts one or more of the following actions: re-evaluation process 404 of its assessment of this variant, flagging 403 the reported cases associated with this variant (i.e. this indicates a review being required before the result can be used in medical diagnostics), sending out e-mail notifications to lab users, and starting the semi-automated conflict resolution workflow.

In another embodiment the central hub component 100 does not alter or initiate the altering of the data in the data repositories 101, 102, 103 but rather stores metadata pertaining to reference data objects centrally in non-transient memory such as information of the final assessment after conflict resolution.

In another embodiment of the claimed subject matter, the re-evaluation process is performed in the central component 100 based on the metadata. Every single step in the automated or semi-automated re-evaluation is documented and stored in the central hub component 100. At any moment in time this process can therefore be audited, reviewed or re-performed.

The data hub component 100 may aggregate information across all data repositories 101, 102, 103. This may be considered in form of a search request issued by one of the data repositories 101, 102, 103 or a reader terminal 104 and submitted to the hub component 100. The hub 100 then forwards the request to the data repositories 101, 202, 103. The hub component 100 is then able to receive search results and return them to the entity initiating the request.

In another embodiment the central hub component 100 performs continuous data maintenance. It is continuously integrating and consolidating new information which would not be possible manually given the size of the data repositories 101, 102, 103. Information is forwarded to one or more data repositories 101, 102, 103 which may be determined by a configuration of the central component 100.

In another embodiment incentives are generated for participating parties (the organizations maintaining data repositories, curators submitting data to repositories etc.). Successful participation in conflict resolution enhances the personal, organisation and/or database-related quality score. The quality score is made public to the network, preferably in form of a "badge" system representing levels of achievement. In this way, participating parties are in-centivised to enhance the quality of data in the entire network. In another embodiment the achievement levels are exposed to 3rd parties such that they can be used to establish an expert reputation.

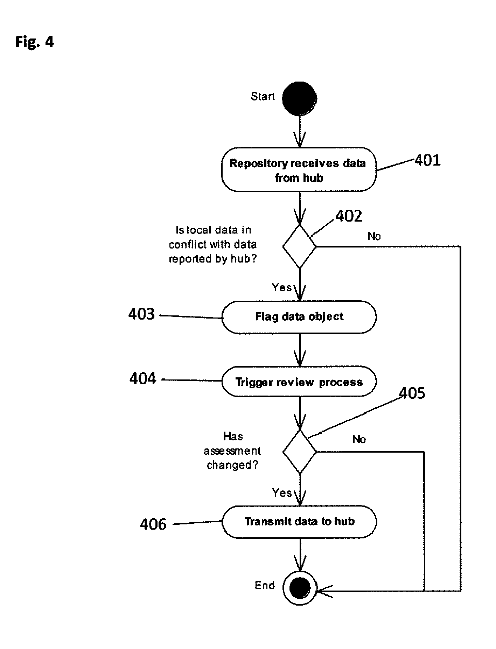

Fig. 4 shows the review process, according to an exemplary embodiment, that is performed by the data repository upon receiving 401 data from the hub 100. The data repository first determines 402 whether there is a conflict between the data stored by the data repository and the hub. In this case the data is flagged 403 and a data review process is triggered 404. Afterwards it is determined 406 whether the assessment has been changed by the review process in which case the updated data is submitted 406 to the hub 100.

In another embodiment the system comprises a data repository which contains biological reference data. The data repository exhibits an interface to a central hub component. The data repository is capable of displaying data which is stored both locally and in the central hub component 100. This is important e. g. when data object attributes differ from the central hub version in the local version. In the case of human DNA variants this could be the classification of a DNA variant which is classified as "benign" locally but as "pathologic" by the central hub component 100.

In another embodiment the local data repository 101, 102, 103 may be configured to overwrite data attributes with data received from the hub 100 if one or more quality scores of the data attributes from the hub 100 is higher than the local score. In another embodiment the local repository 101, 102, 103 supports data entry and curation as an independent process which is subject to change and which needs to be documented formally. The entities operating data repositories 101, 102, 103 may have different requirements on the details and documentation of these processes. By separating the process definition from the implementation of the software both changes of processes and changes/updates of software are de-coupled and can be performed independently.

In another embodiment the local repository 101, 102, 103 provides modules which comprise one or more steps of a workflow which can be used to construct an entire workflow for data entry and review. Another quality score can be derived from the structure of these workflows: an entity that deploys a workflow comprising specific steps receives a higher score than an entity that deploys a workflow with only a subset of steps. Similarly a quality score may be related to a data object that was created following a specific workflow.

In another embodiment the local data repository 101, 102, 103 mediates reviews and re-assessments of data objects by a workflow comprising displaying a list of conflicting data, displaying data attributes received from the central hub component 100 and providing means to enter additional information and send additional information to the central hub component 100.

In another embodiment the local data repository 101, 102, 103 de-identifies all data that is transmitted to the central hub component 100.

In another embodiment the local data repository 101, 102, 103 displays information received from the central hub component 100 during data entry before data is committed to the local data repository storage 206. Preferably the information displayed relates to potential conflicts with data objects registered with the central hub component 100.

In another embodiment, additional data repositories 101, 102, 103 are provided to represent publicly available data sets. These special data repositories 101, 102, 103 can be updated on a regular basis, by using the data via the data and model transformation approach as described above. Users can thus consider the reference data with which they may disagree, expressed in the same nomenclature (and user interface) as other data from the system.

Data, which has been updated and improved by the review process, can be used as input for automated applications, clinical applications and/or industrial processes and can, thus, be used to improve other processes and/or to make other processes more cost, time and/or energy efficient.

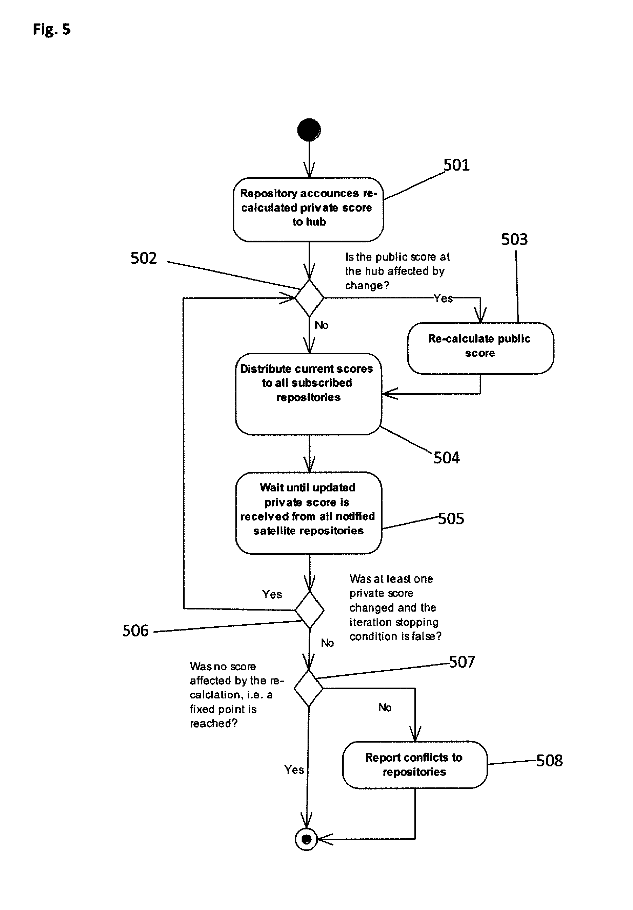

In another embodiment, as shown in Fig. 5, each data repository 101, 102, 103 can also compute and distribute its own quality scores, which may be based on the quality scores of the hub 100 and the other data repositories 101, 102, 103, as well as on data that otherwise could not be used for ethical or legal reasons (because this would imply that the data is sent to the hub). Data repositories 101, 102, 103 can assign a weighting factor for the quality scores coming from the host and from other data repositories, and thereby create a "network of trust".

Fig. 5 shows an exemplary workflow for managing the distributed computation of (private) quality scores as controlled by the hub 100. Repositories 101, 102, 103 may define their private quality scores by relying on the private scores of other repositories 102, 102, 103, thereby implicitly subscribing to quality score changes in those repositories 101, 102, 103. A data repository 101, 102, 103 then announces 501 a re-calculated private score to the hub 100. The hub 100 determines 502 if the public score is affected by the change and, in this case, re-calculates 503 the public score. Then the hub 100 distributes 504 the current scores to all subscribing repositories, causing these to re-calculate their private score, which are then received 505 by the hub 100. As this may introduce cyclic dependencies between private quality scores, the re-calculation is executed iteratively. The stopping condition 506 for the iterative computation could, for example, only allow a fixed number of iterative re-calculations, or it could stop the recalculation whenever the differences after re-calculation are negligible. In case conflicting scores cannot be resolved by such an iterative re-calculation 507, manual, semi-automated or automated conflict resolution is triggered and the conflicts are reported 508 to the repositories 101, 102, 103. The hub 100 may trigger a distributed re-computation of the quality score by querying the data repositories 101, 102, 103, e. g. in case new information on a set of matching data objects is available.

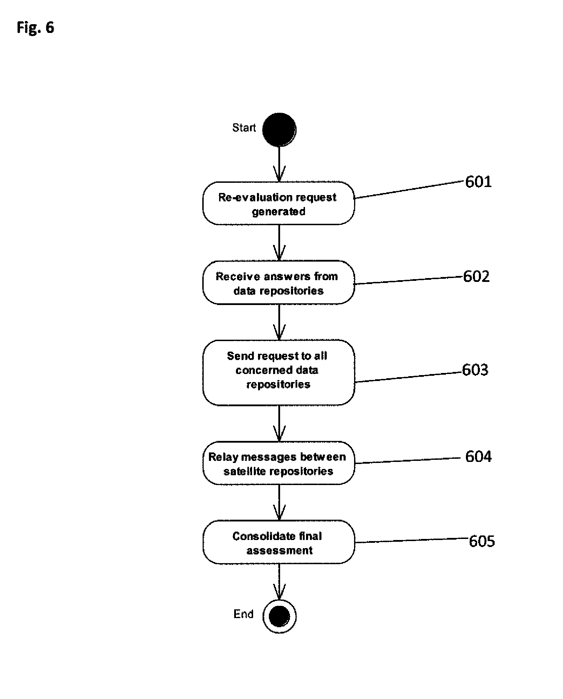

In another embodiment, as shown in Fig. 6 the re-evaluation process may be mediated by the hub component 100. The hub 100 initializes a specific workflow for re-evaluation of data. Such a workflow may comprise:

- Receiving a re-evaluation request 601 from a data repository 101, 102, 103 or a reader terminal 104. Alternatively the central hub component 100 may issue a re-evaluation request itself on discovery of a data conflict.
- Receiving answers 602 from data repositories 101, 102, 103,
- Sending a request to review 603 a specific data object to all data repositories 101, 102, 103 concerned,
- Mediating a semi-automated conflict resolution by relaying messages 604 between data repositories 101, 102, 103, such messages potentially containing additional data supporting or contradicting a specific data attribute,
- Consolidating and storing 605 a final assessment of the data object attribute.

While the workflow described above may be applicable to smaller data repositories 101, 102, 103 with slowly changing content, the claimed subject matter provides faster and more automated workflows for large and quickly changing data repositories. In one embodiment the hub component 100 computes a quality score for the data object according to the metadata stored with the data object in the data repository. The hub 100 then compares the quality scores of the data objects from different data repositories and automatically chooses the attribute of the highest ranking data object as final assessment.

For the following examples, let $c_1, ...,c_n$ be all clinical cases of a data repository 101, 102, 103 that are associated with a specific variant, and let each case $c_i$ consist of k meta-data attributes: $c_i = (d^i_1,...,d^i_k)$. Among other things the following information is considered meta-data: experimental data or evidence supporting the classification of the data object, information about samples, subjects, experimental or clinical history of subjects. In a simplified embodiment the quality score q is a linear function of the number of metadata objects related to the data object in question, e.g.

$$q = an + b$$

More elaborate quality scores may use a weighted function of related metadata where the weight $w_j$ of the metadata depends on its type:

$$q = \sum_{i=1}^{n} q_i, \text{ with } q_i = \sum_{j=1}^{k} w_j \cdot d_j^i$$

Metadata that may be considered contributing strongly to the quality score may be e. g. experimentally measured data (= quantitative data). Qualitative data on the other hand may be considered of less importance for the quality score. A quality score may also be determined by the consistency of the metadata that is related to a specific data object. Inconsistent metadata will therefore lower the quality score and vice versa.

In another embodiment the statistical distribution of classifications of data objects - if several of these classifications exist in the central hub 100 - is determined by the data repository network. The central hub component 100 then determines, e.g. computes, the mean or median or another meaningful parameter of the distribution and uses the result to determine the final assessment to resolve the conflict in classification. In a further development a weighting is applied to the values in the statistical distribution according to a score W attributed to the specific data repository 201, 102, 103 or the specific human or automated curator who submitted the data to the data repository, e.g.:

$$q = W \cdot \sum_{i=1}^{n} q_i,$$

with $q_i$ defined as above.

In another embodiment quality scores are determined from properties of the data repository or properties of specific parts of the repository or the organization which is maintaining the repository. Larger repositories or repositories with a high data generation rate may be attributed a higher score globally. Quality scores may also be derived from properties of specific sub-domains of a repository. A specific repository may contain e. g. many datasets related to a specific gene so that this specific repository may be rated to have expert knowledge in that domain. When comparing a data object from that sub-domain to a corresponding data object from another repository the repository with the higher number of datasets may be attributed a higher quality score and therefore classifications and data attributes from this repository may be preferred over other repositories. Instead of the number of datasets also other parameters $p_1, ..., p_l$ may be used to determine quality scores like a number of subjects which were examined in a sub-domain or number of biological objects (e. g. DNA variants) that were found in a sub-domain, e.g.

$$q = W(p_1, ..., p_l) \cdot \sum_{i=1}^{n} q_i,$$

with $q_i$ defined as above.

In another embodiment the factors used in the quality scoring method are adaptively re-weighted, by monitoring the predictive power of each kind of metadata on specific data objects, and how it changes over time. This allows to continuously improve also the quality scoring method itself, e.g. to identify the waning (or gaining) impact of the lab reputation or the number of similar data objects in a given data repository as a measure of its trustworthiness.

In another embodiment the history of re-evaluations in which a certain entity (repository/organisation/curator) was involved is used to compute a quality score. An entity whose data assessments historically prevailed in re-evaluations will be preferred over other entities.

In another embodiment the hub component 100 can perform model transformations between the data models from the data repositories 101, 102, 103 such that it is capable of mapping the data models of the data repositories as well as their ontologies onto each other. As an example this may be applied e. g. to mapping of DNA variants of the human genome. The nomenclature to describe variants in the human genome is not bijective. This means that a specific variation may validly be described by two different terms. The hub component may apply a stricter, non-ambiguous nomenclature and apply a transformation to all data objects from data repositories accordingly. Another example of ontology mapping is the mapping of different DNA variant classifications. Every data repository entity may define its own classification scheme to rate variants in the human genome, which may deviate from recommendations as set forth by e. g. the American College of Medical Genetics and Genomics. In order to correctly compare and match DNA variants from different data repositories the hub component applies transformations to the data repository classification schemes into its own classification ontology.

Since data models and ontologies are subject to continuous change the hub component allows for changes in the data model and ontology transformations. To this end only the specific module of the hub component must be updated or exchanged that is responsible of the model transformation for the specific data repository 101, 102, 103. The central hub component 100 maintains two different interfaces to the data repositories: one dedicated to the exchange of biological reference data, the other dedicated to the exchange of information regarding models and ontologies.

List of references:

| | |
|---|---|
| 100 | central computing component |
| 101 | data repository |
| 102 | another data repository |
| 103 | another data repository |
| 104 | reader terminal |
| 201 | communication module of central computing component |
| 202 | quality score module of central computing component |
| 203 | storage module of central computing component |
| 204 | model transformation module of central computing component |
| 205 | metadata module of data repository |
| 206 | storage module of data repository |
| 207 | communication module of data repository |
| 208 | data management module of data repository |
| 301 | transmitting data to central computing component by data repository |
| 302 | determining if there are similar objects in the network |
| 303 | retrieving similar objects from storage |
| 304 | calculating quality score by central computing component |
| 305 | storing quality score by central computing component |
| 306 | transmitting quality score to one or more data repository |
| 401 | receiving data from central computing component by data repository |
| 402 | determining data conflict |
| 403 | flagging data object |
| 404 | triggering review process |
| 405 | determining change in assessment |
| 406 | transmitting data to central computing component |
| 501 | announcing re-calculated private score to central computing component |
| 502 | determining if public score is affected by change |
| 503 | recalculating public score |
| 504 | distributing current scores to subscribing data repositories |
| 505 | receiving updated private scores from data repositories |
| 506 | determining if at least one private score was changed and the stopping iteration condition is false |
| 507 | determining if no score was affected, i.e. a fixed point is reached |
| 508 | reporting conflicts to repositories |
| 601 | generating re-evaluation request |
| 602 | receiving answers from data repositories |
| 603 | sending requests to concerned data repositories |
| 604 | relaying messages between satellite repositories |
| 605 | consolidating final assessment |

This application relates, in accordance with the examples and with the addition of further aspects, to the following aspects. The applicant reserves the right to file future divisional applications according to any part and combination of the subject matter of the description as well as the aspects.

**System according to central computing component**

1. A data quality management system comprising

- a central computing component, implemented on a computing device, comprising a computer-implemented data storage module, a computer-implemented data communication module and a computer-implemented quality score module; and
- computer-implemented data transmission connections to a first and a second computer implemented data repository stored on at least one database server;

wherein the central computing component is configured to receive, via the communication module, a first data point comprising a first obtained data and a first assigned value from the first data repository, to determine, in the quality score module, a first quality score of the first data point, to determine a first storable data from the first data point and/or the first quality score and to store the first storable data in the storage module;
wherein the central computing component is further configured to receive, via the computer-implemented communication module, a second data point comprising a second obtained data and a second assigned value from the second data repository, to determine, in the quality score module, a second quality score of the second data point, to determine a second storable data from the second data point and/or the second quality score and to store the second storable data in the storage module; and wherein the second obtained data is similar to the first obtained data according to a predefined similarity measure and the central computing component is further configured to transmit a second transmittable data, determined from the second data point and/or the second quality score to the first data repository, causing the first data repository to re-evaluate the first assigned value.

2. The system according to aspect 1, wherein the central component is further configured to transmit the first quality score to the first data repository and/or to transmit the second quality score to the second data repository.

3. The system according to aspect 1 or 2, wherein the central computing component is configured to transmit the second transmittable data to the first data repository causing the first data repository to update the first assigned value.

4. The system according to aspect 3, wherein the central computing component is further configured to receive an updated first data point comprising the first obtained data and an updated first assigned value from the first data repository, to determine, in the quality score module, an updated first quality score of the updated first data point, to determine an updated first storable data from the updated first data point and/or the updated first quality score, to store the updated first storable data in the storage module.

5. The system according to aspect 4, wherein the central computing component is further configured to transmit, via the computer-implemented data communication module, the updated first quality score to the first and/or the second data repository.

6. The system according to aspect 4 or 5, wherein the updated first assigned value is different from the first assigned value.

7. The method according to any of the preceding aspects, wherein the first assigned value, the second assigned value, the first quality score and/or the second quality is a vector comprising at least two distinct values.

8. The system according to any of the preceding aspects, wherein the first assigned value and/or the second assigned value comprises at least one expert opinion.

9. The system according to any of the preceding aspects, wherein the storable data determined from a received data point and/or a corresponding quality score comprises at least one of information about the data repository which the received data was received from, a time stamp, a unique identifier and the quality score.

10. The system according to any of the preceding aspects, wherein the first and/or the second obtained data comprises biological, medical and/or genomic data.

11. The system according to any of the preceding aspects, wherein the first assigned value and/or the second assigned value further comprises a confidence score.

12. The system according to any of the preceding aspects, further comprising a computer-implemented model transformation module, wherein the first data repository contains data in a first data format and the second data repository contains data in a second data format and the central component is further configured to transform, in the data transformation module, data received from the first data repository into the second data format, data received from the second data repository into the first data format and/or data received from the first and/or second data repository into a central data format.

13. The system according to any of aspects 4 to 12, wherein the central component is further configured to overwrite the first storable data with the updated first storable data.

14. The system according to any of aspects 4 to 12, wherein the central component is further configured to keep the first storable data in the storage module when storing the updated first storable data, so as to create a history of data updates.

15. The system according to any of the preceding aspects, wherein the quality score module comprises at least one adaptive parameter, which is used to determine at least one of the first quality score and the second quality score.

16. The system according to aspect 15, wherein at least one of the at least one adaptive parameters is determined by the quality score module based on a statistical distribution of at least some data stored in the storage module.

17. The system according to any of aspects 1 to 16, wherein the system further comprises at least one of a first and/or a second computer implemented data repository interface configured to be run on a data base server, wherein the data repository interface is configured to transmit the first data point comprising the first obtained data and the first assigned value to the central computing component, to receive information about the second data point from the central computing component and to re-evaluate and/or cause the data repository to re-evaluate the first assigned value on the basis of the received information about the second data point.

18. The system according to aspect 17, wherein the first and/or the second computer implemented data repository interface is further configured to receive and store, in the data repository, a first quality score of the first data point and/or to receive a second quality score of the second data point from the central computing component.

19. The system according to aspect 17, wherein the computer-implemented data repository interface is further configured to determine a quality score of a data point stored in the data repository or received from the central computing component or another data repository.

20. The system according to any of aspects 18 or 19, wherein the first assigned value is re-evaluated on the basis of the received information about the second data point and the received and/or determined quality scores.

21. The system according to any of aspects 17 to 20, wherein the data repository interface is further configured to update the first assigned value, on the basis of the received information about the second data point, to an updated first assigned value different from the first assigned value.

22. The system according to any of the preceding aspects, wherein the first obtained data comprises metadata relating to data stored in the data repository.

23. The system according to aspect 22, wherein the metadata comprises data relating to a number of similar instances stored in the data repository.

24. The system according to any of the preceding aspects further comprising at least one of the first and/or the second data repository.

**Main method according to central component**

25. A method for automatic data quality management, comprising the following steps, implemented to be executed on a computer processor:

- receiving a first data point comprising a first obtained data and a first assigned value from a first data repository,
- determining a first quality score of the first data point,
- determining a first storable data from the first data point and/or the first quality score,
- storing the first storable data in a computer implemented central storage module,
- receiving a second data point comprising a second obtained data, which is similar to the first obtained data according to a predefined similarity measure, and a second assigned value from a second data repository,
- determining a second quality score of the second data point,
- determining a second storable data from the second data point and/or the second quality score,
- storing the second storable data in the storage module and
- transmitting a transmittable second data determined from the second data point and/or the second quality score to the first data repository causing the first data repository to re-evaluate the first assigned value.

26. The method according to aspect 25, further comprising the step of transmitting the first quality score to the first data repository and/or transmitting the second quality score to the second data repository.

27. The method according to aspect 25 or 26, wherein transmitting the transmittable second data to the first data repository causes the first data repository to update the first assigned value.

28. The method according to any of aspects 25 to 27 further comprising the steps of

- receiving an updated first data point comprising the first obtained data and an updated first assigned value from the first data repository,
- determining an updated first quality score of the updated first data point,
- determining an updated first storable data from the updated first data point and/or the updated first quality score, and
- storing the updated first storable data in the central storage module.

29. The method according to any of aspects 25 to 28 further comprising the step of transmitting the updated first quality score to the first and/or the second data repository.

30. The method according to any of aspects 27 to 29 wherein the updated first assigned value is different from the first assigned value.

31. The method according to any of aspects 25 to 30, wherein the quality scores are determined by statistical methods involving weighting, according to weighting parameters, of the obtained data, and/or determining a mean or a median value of the obtained data.

32. The method according to any of aspects 25 to 31, wherein the first assigned value, the second assigned value, the first quality score and/or the second quality score is a vector comprising at least two distinct values.

33. The method according to any of aspects 25 to 32, wherein the first assigned value and/or the second assigned value comprises at least one expert opinion.

34. The method according to any of aspects 25 to 33, wherein the first and/or the second obtained data comprises biological, medical and/or genomic data.

35. The method according to any of aspects 25 to 34, wherein the first assigned value and/or the second assigned value further comprises a confidence score.

36. The method according to any of aspects 28 to 35, wherein the first storable data is overwritten by the updated first storable data.

37. The method according to any of aspects 25 to 35, wherein the first storable data is kept in a memory when storing the updated first storable data, so as to create a history of data updates.

38. The method according to any of aspects 25 to 36, wherein determining at least one of the first quality score and the second quality score is based on at least one adaptive parameter.

39. The method according to aspect 38, wherein at least one of the at least one adaptive parameters is determined based on a statistical distribution of at least some data stored in the memory.

## Computer Program Product

40. A computer program product for data quality management stored on a computer readable medium which, when run on a computer, is configured to execute the method of any of aspects 25 to 39.

## Method according to data repository

41. A method for automatically improving data quality of a computer-implemented data repository involving the following steps:

- transmitting a first data point comprising a first obtained data and a first assigned value to a central computing component
- receiving information about a second data point comprising a second obtained data, which is similar to the first obtained data according to a predefined similarity measure, and a second assigned value from the central computing component
- re-evaluating the first assigned value on the basis of the received information about the second data point.

42. The method of aspect 41, wherein the method further involves the step of receiving and storing, in the data repository, a first quality score of the first data point and/or receiving a second quality score of the second data point from the central computing component.

43. The method according to aspect 41 or 42, wherein the method further comprises the step of determining quality scores of a data point stored in the data repository or received from the central computing component or another data repository.

44. The method according to any of aspects 41 to 43, wherein the first assigned value is re-evaluated on the basis of the received information about the second data point and the received and/or determined quality scores.

45. The method according to any of aspects 41 to 44, wherein re-evaluating the first assigned value includes updating the first assigned value to an updated first assigned value different from the first assigned value.

46. The method according to any of aspects 41 to 45 wherein the first obtained data comprises metadata relating to data stored in the database.

47. The method of aspect 46, wherein the metadata comprises data relating to a number of similar instances stored in the data repository.

## System including data repository interface

48. The system according to any of aspects 1 to 16, wherein the system further comprises at least one of a first and/or a second computer implemented data repository interface configured to be run on a data base server, wherein the data repository interface is configured according to any of aspects 41 to 47.

## Claims

1. A data quality management system comprising

  - a central computing component (100), implemented on a computing device, comprising a computer-implemented data storage module (203), a computer-implemented data communication module (201) and a computer-implemented quality score module (202); and
  - computer-implemented data transmission connections (105, 106) to a first and a second computer implemented data repository (101, 102) stored on at least one database server;

wherein the central computing component (100) is configured to receive, via the communication module (201), a first data point comprising a first obtained data and a first assigned value from the first data repository (101), to determine, in the quality score module (202), a first quality score of the first data point, to determine a first storable data from the first data point and/or the first quality score and to store the first storable data in the storage module (203); wherein the central computing component (100) is further configured to receive, via the computer-implemented communication module (201), a second data point comprising a second obtained data and a second assigned value from the second data repository (102), to determine, in the quality score module (202), a second quality score of the second data point, to determine a second storable data from the second data point and/or the second quality score and to store the second storable data in the storage module (203); wherein the second obtained data is similar to the first obtained data according to a predefined similarity measure and the central computing component (100) is further configured to transmit a second transmittable data, determined from the second data point and/or the second quality score to the first data repository (101), causing the first data repository (101) to re-evaluate the first assigned value.

2. The system according to claim 1, wherein the central computing component (100) is configured to transmit the second transmittable data to the first data repository (101) causing the first data repository (101) to update the first assigned value.

3. The system according to claim 2, wherein the central computing component (100) is further configured to receive an updated first data point comprising the first obtained data and an updated first assigned value from the first data repository (101), to determine, in the quality score module (202), an updated first quality score of the updated first data point, to determine an updated first storable data from the updated first data point and/or the updated first quality score, to store the updated first storable data in the storage module (203).

4. The system according to claim 3, wherein the central computing component (100) is further configured to transmit, via the computer-implemented data communication module (201), the updated first quality score to the first and/or the second data repository (101, 102).

5. The system according to any of the preceding claims, further comprising a computer-implemented model transformation module (204), wherein the first data repository (101) contains data in a first data format and the second data repository (102) contains data in a second data format and the central component (100) is further configured to transform, in the data transformation module (204), data received from the first data repository (101) into the second data format, data received from the second data repository (102) into the first data format and/or data received from the first and/or second data repository (101, 102) into a central data format.

6. The system according to any of the preceding claims further comprising at least one of the first and/or the second data repository (101, 102).

7. A method for automatic data quality management, comprising the following steps, implemented to be executed on a computer processor:

   - receiving (301) a first data point comprising a first obtained data and a first assigned value from a first data repository (101),
   - determining (304) a first quality score of the first data point,
   - determining a first storable data from the first data point and/or the first quality score,
   - storing (306) the first storable data in a computer implemented central storage module (203),
   - receiving (301) a second data point comprising a second obtained data, which is similar to the first obtained data according to a predefined similarity measure, and a second assigned value from a second data repository (102),
   - determining (304) a second quality score of the second data point,
   - determining a second storable data from the second data point and/or the second quality score,
   - storing the second storable data in the storage module (203) and
   - transmitting a transmittable second data determined from the second data point and/or the second quality score to the first data repository causing the first data repository (101) to re-evaluate the first assigned value.

8. The method according to claim 7, wherein transmitting the transmittable second data to the first data repository causes the first data repository (101) to update the first assigned value.

9. The method according to any of claims 7 or 8 further comprising the steps of

   - receiving an updated first data point comprising the first obtained data and an updated first assigned value from the first data repository (101),
   - determining (304) an updated first quality score of the updated first data point,
- determining an updated first storable data from the updated first data point and/or the updated first quality score, and
- storing the updated first storable data in the central storage module (203).

10. The method according to any of claims 8 to 9 further comprising the step of transmitting the updated first quality score to the first and/or the second data repository (101, 102).

11. The system or the method according to any of the preceding claims, wherein the first and/or the second obtained data comprises biological, medical and/or genomic data.

12. A computer program product for data quality management stored on a computer readable medium which, when run on a computer, is configured to execute the method of any of claims 7 to 11.

13. A method for automatically improving data quality of a computer-implemented data repository (101) involving the following steps:

- transmitting (301) a first data point comprising a first obtained data and a first assigned value to a central computing component (100),
- receiving information about a second data point comprising a second obtained data, which is similar to the first obtained data according to a predefined similarity measure, and a second assigned value from the central computing component (100),
- re-evaluating the first assigned value on the basis of the received information about the second data point.

14. The method according to claim 13, wherein the method further comprises the step of determining a quality score of a data point stored in the data repository (101) or received from the central computing component (100) or another data repository (102).

15. The system according to any of claims 1 to 6, wherein the system further comprises at least one of a first and/or a second computer implemented data repository interface configured to be run on a data base server, wherein the data repository interface is configured according to any of claims 13 to 14.

**Fig. 1**

**Fig. 2**

**Fig. 3**

Start ●

Repository "A"
transmits data to hub — 301

Hub determines if there are
corresponding objects in the
network

302

303

Hub retrieves quality
score for corresponding
objects from storage

Hub calculates
repository "A" quality
scores — 304

Hub stores quality
scores —305

Hub transmits quality
scores to one or more
repositories —306

End ◉

**Fig. 4**

Start ●

Repository receives data
from hub ───401

Is local data in
conflict with data
reported by hub?

402

No

Yes

403 ── Flag data object

404 ── Trigger review process

405

Has
assessment
changed?

No

Yes

406 ── Transmit data to hub

End ●

**Fig. 5**

Repository accounces re-
calculated private score
to hub — 501

Is the public score at
the hub affected by
change?

502

503

Yes

No

Re-calculate public
score

Distribute current scores
to all subscribed
repositories

504

Wait until updated
private score is
received from all notified
satellite repositories — 505

Was at least one
private score
changed and the
iteration stopping
condition is false?

Yes

506

No

Was no score
affected by the re-
calclation, i.e. a
fixed point is
reached?

507

No

Report conflicts to
repositories — 508

Yes

**Fig. 6**

Start ●

Re-evaluation request generated —— 601

Receive answers from data repositories —— 602

Send request to all concerned data repositories —— 603

Relay messages between satellite repositories —— 604

Consolidate final assessment —— 605

End ◉

Europäisches
Patentamt

European
Patent Office

Office européen
des brevets

# EUROPEAN SEARCH REPORT

Application Number

EP 15 17 0816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/027965 A1 (GARRETT ANDREW J [GB] ET AL) 31 January 2008 (2008-01-31)<br>* abstract *<br>* paragraph [0018] - paragraph [0035] *<br>* paragraph [0007] *<br>----- | 1-15 | INV.<br>G06F19/28<br>G06F17/30 |
| X | US 2014/222966 A1 (MARINS SERGEJS [CA] ET AL) 7 August 2014 (2014-08-07)<br>* abstract *<br>* paragraph [0010] - paragraph [0024] *<br>* paragraph [0038] - paragraph [0074] *<br>* paragraph [0085] - paragraph [0107] *<br>* paragraph [0118] - paragraph [0137] *<br>* paragraph [0141] - paragraph [0169]; figures 1,2,4 *<br>* paragraph [0265] *<br>----- | 1-15 | |
| A | US 2004/186842 A1 (WESEMANN DARREN [US]) 23 September 2004 (2004-09-23)<br>* abstract *<br>* paragraph [0018] - paragraph [0024] *<br>* paragraph [0032] - paragraph [0062] *<br>* claims 1-5,11-15 *<br>----- | 1-15 | |
| A | US 6 141 664 A (BOOTHBY DAVID J [US]) 31 October 2000 (2000-10-31)<br>* abstract *<br>* column 3, line 66 - column 7, line 14 *<br>* column 9, line 9 - column 11, line 53 *<br>* column 15 *<br>----- | 1-15 | |

TECHNICAL FIELDS
SEARCHED      (IPC)

G06F

-/--

The present search report has been drawn up for all claims

| Place of search | Date of completion of the search | Examiner |
|---|---|---|
| Munich | 13 November 2015 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 15 17 0816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PILAR A ET AL: "Detection and Resolution of Data Inconsistencies and Data Integration using Data Quality Criteria", INTERNET CITATION, 2004, XP002392215, Retrieved from the Internet: URL:http://www.macs.hw.ac.uk/~pilar/resear ch/QuaTIC.pdf [retrieved on 2006-07-26] * abstract * * page 1, right-hand column, paragraph 3 - page 2, left-hand column, paragraph 2 * * page 4, right-hand column, paragraph 2 - paragraph 4 * | 1-15 | |

-----

TECHNICAL FIELDS
SEARCHED      (IPC)

The present search report has been drawn up for all claims

| Place of search | Date of completion of the search | Examiner |
|---|---|---|
| Munich | 13 November 2015 | Kürten, Ivayla |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

         

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 15 17 0816

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2015

| Patent document cited in search report | | Publication date | Patent family member(s) | | Publication date |
|---|---|---|---|---|---|
| US 2008027965 | A1 | 31-01-2008 | US 2008027965 A1<br>US 2013132354 A1 | | 31-01-2008<br>23-05-2013 |
| US 2014222966 | A1 | 07-08-2014 | NONE | | |
| US 2004186842 | A1 | 23-09-2004 | US 2004186842 A1<br>WO 2004084002 A2 | | 23-09-2004<br>30-09-2004 |
| US 6141664 | A | 31-10-2000 | US 6141664 A<br>US 6532480 B1 | | 31-10-2000<br>11-03-2003 |

EPO FORM P0459

For more details about this annex : see Official Journal of the European Patent Office, No. 12/82

**EP 3 101 574 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8359297 B